# EUROPÄISCHE PATENTSCHRIFT

(11) **EP 1 368 040 B1**
(45) Veröffentlichungstag und Bekanntmachung des Hinweises auf die Patenterteilung: **05.07.2006**
(21) Anmeldenummer: 02701291.3
(22) Anmeldetag: 22.02.2002
(51) Int. Cl.: A61K 31/7072, A61K 31/4706, A61P 33/00

(54) **VERWENDUNG VON 5-SUBSTITUIERTEN NUKLEOSIDEN UND/ODER DEREN PRODRUGS ZUR RESISTENZFREIEN THERAPIE VON INFEKTIONSKRANKHEITEN**
USE OF 5-SUBSTITUTED NUCLEOSIDES AND/OR PRODRUGS THEREOF IN THE RESISTANCE-FREE TREATMENT OF INFECTIOUS DISEASES
UTILISATION DE NUCLEOSIDES 5-SUBSTITUES ET/OU DE LEURS PROMEDICAMENTS POUR TRAITER DES MALADIES INFECTIEUSES SANS INDUIRE DE RESISTANCE

(30) Priorität: 23.02.2001 DE 10108851
(43) Veröffentlichungstag der Anmeldung: 10.12.2003
(73) Patentinhaber: Resprotect GmbH, 01307 Dresden (DE)
(72) Erfinder: FAHRIG, Rudolf, Hinrich, Hermann, 30657 Hannover (DE); SONNTAG, Denise, 01309 Dresden (DE)
(74) Vertreter: Pfenning, Meinig & Partner GbR
(86) Internationale Anmeldenummer: PCT/EP2002/001890
(87) Internationale Veröffentlichungsnummer: WO 2002/067951

(56) Entgegenhaltungen:
- DE-A- 19 545 892
- US-A- 5 763 447
- PANCHEVA, S. N.: "Methotrexate potentiates anti-herpes simplex virus type 1 activity of E-5-(2-bromovinyl)-2'- deoxyuridine." ACTA VIROLOGICA, (1995) VOL. 39, NO. 2, PP. 117-119. , XP008010809

## Beschreibung

Die Resistenzbildung gegenüber der Behandlung mit Medikamenten stellt einen universellen Abwehrmechanismus von Mikroorganismen, Tieren und Pflanzen dar. Beim Menschen findet man diesen Abwehrmechanismus in Tumoren. Meist führt die Vervielfachung (Amplifikation) bestimmter Gene zur Resistenzbildung. Diese Genamplifikation verursacht die Überproduktion eines Genprodukts, das direkt oder indirekt die Wirkung des Arzneimittels reduziert. Die Resistenzbildung von Plasmodien (Erreger der Malaria) und Leishmanien (Flagellaten als Erreger der Orientbeule u.a.m.) beruht teilweise auf der Amplifikation der gleichen Gene wie in menschlichen Tumoren.

Der Nachweis, daß Genamplifikation bei Bakterien zu Resistenzen führt, ist bei Proteusbakterien, Escherichia coli, Streptokokken und Staphylokokken gelungen. Alle sind wichtige Hospitalismuskeime und Erreger von Harnweginfekten (Romero and Palacios, gene amplification und genome plasticity in procaryotes, Ann. Rev. Genet. 1997). Der der Genamplifikation zugrunde liegende Mechanismus, die Rekombination, kann ebenfalls zur Resistenzbildung führen. Eindeutig nachgewiesen ist diese Form der Resistenzbildung ausnahmslos bei allen Arten von Bakterien, jedoch auch für menschliche Tumoren und alle bisher untersuchten Organismen.

Malaria ist eine Sammelbezeichnung für Infektionen durch Protozoen der Gattung Plasmodium. Durch zunehmende Resistenz der Plasmodien gegen Chemotherapeutika und der Anopheles-Mücken gegen Insektizide verschlechtert sich die Situation zunehmend. Beide Resistenzen können durch Genamplifikation/Rekombination verursacht werden.

Als Antimalaria-Arzneimittel werden 4-Aminochinoline (Chloroquin, Amodiaquin, Mepacrin und Sontaquin) verwendet. Diese sind Analoga von Chinin. Weiterhin gibt es zwei Gruppen von Antifolaten, einmal die Dihydrofolat-Reduktase (DHFR) -Inhibitoren (Pyrimethamin und Proguanil) und zum anderen die Sulphone und Sulphonamide. Resistenzen können daher durch Amplifikation des DHFR-Gens entstehen (Cowman and Lew, 1989; Cowman and Lew, 1990; Tanaka et al., 1990a; Tanaka et al. 1990b; Watanabe and inselburg, 1994). Die Amplifikation von "Multi-drug-resistance"-Genen spielt ebenfalls eine Rolle (Foote et al., 1989). Die Amplifikation des pfmdrl-Gens ist z.B. an die Resistenz gegenüber Mefloquin, Halofantrin und Chinin gebunden (Wilson et al., 1989; Cowman et al., 1994). Durch zunehmende Resistenz der Plasmodien gegen Chemotherapeutika und der Anopheles-Mücken gegen Insektizide verschlechtert sich die Situation zunehmend. Beide Resistenzen können durch Genamplifikation/Rekombination verursacht werden. Über die Verhinderung Chemotherapie-induzierter Genamplifikation soll die Resistenzbildung von Plasmodien verhindert werden.

Leishmaniasen sind durch Leishmanien (intrazellulär parasitierende Protozoen der Klasse Mastigophora) verursachte und durch Phlebotomen (Sandmücken) übertragene Infektionskrankheiten. Resistenzen gegenüber einer Chemotherapie beruhen auf der Amplifikation einiger Gene, die auch bei der Chemoresistenz von Tumoren eine Rolle spielen (Ouellette and Borst, 1991; Grondin et al., 1998; Arana et al., 1998; Heimeur and Ouellette, 1998; Kundig et al., 1999). Über die Verhinderung Chemotherapie-induzierter Genamplifikation/Rekombination soll die Resistenzbildung verhindert werden.

Die Verwendung von 5-substituierten Nukleosiden zur Hemmung der Resistenzbildung bei der Zytostatikabehandlung ist bereits aus der DE 195 45 892 bekannt.

In Pancheva, S. N.: "Methotrexate potentiates antiherpes simplex virus type 1 activity of E-5-(2-bromvinyl)-2'-deoxyuridine", Acta Virologica (1995) Vol. 39, No. 2., PP. 117-119, ist die Potenzierung der Anti-Herpes-Simplex-Virus-Typ-1-Aktivität beschrieben.

Weiterhin offenbart die US 5,763,447 eine Methode zur Behandlung von Pneumonien mit 5-substituierten Nukleosiden

Aufgabe der vorliegenden Erfindung ist es daher, eine resistenzfreie Behandlung von durch Bakterien oder Protozoen hervorgerufenen Infektionskrankheiten zu ermöglichen.

Diese Aufgabe wird durch die gattungsgemäße Verwendung mit den Merkmalen des Anspruchs 1 gelöst. Die weiteren Unteransprüche 2 bis 19 zeigen vorteilhafte Weiterbildungen auf.

Eine resistenzfreie Therapie von Infektionskrankheiten aus der Gruppe bakterieller oder durch Protozoenhervorgerufenen Infektionen wird dadurch erreicht, daß zusammen mit dem gegen die Infektionskrankheit aktiven Wirkstoff 5-substituierte Nukleoside und/oder deren Prodrugs verabreicht werden.

Dabei können der aktive Wirkstoff und die 5-substituierten Nukleoside bzw. deren Prodrugs sowohl in einer einzigen Formulierung als auch als Kombinationspräparat in getrennten Formulierungen vorliegen. Dabei kann eine gleichzeitige, getrennte oder zeitlich abgestufte Anwendung vorliegen.

Gemäss der Erfindung sind die 5-substituierten Nukleoside ausgewählt aus (E)-5-(2-Bromovinyl)-2-deoxyuridine (BVDU), dessen Salze, dessen Schutzformen und dessen Prodrugs. Als Prodrug kann dabei vorzugsweise die Verbindung der Formel I eingesetzt werden.

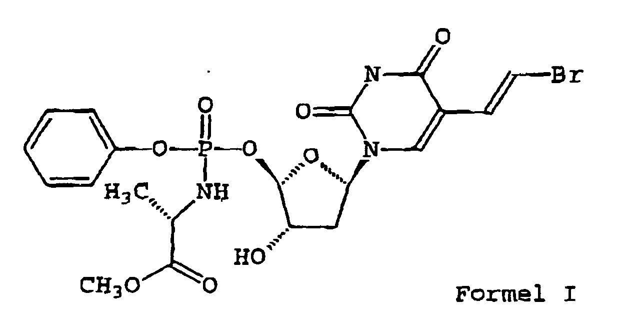

Als aktive Wirkstoffe zur resistenzfreien Therapie bakterieller Infektionskrankheiten werden in einer bevorzugten Ausführungsform Antibiotika eingesetzt. Als die Infektion auslösende Bakterien kommen dabei wegen der erwiesenen Bedeutung der Rekombination bei der Resistenzbildung unterschiedslos alle Bakterien in Frage und wegen der nachgewiesenen Bedeutung der Genamplifikation z.B. Proteusbakterien, Escherichia coli, Streptokokken und Staphylokokken in Frage.

Für die resistenzfreie Therapie von Malaria oder anderer durch Plasmodien verursachter Infektion werden als aktive Wirkstoffe Antibiotika und/oder Antiinfektiva wie z.B. 4-Aminochinoline eingesetzt. Besonders bevorzugt werden dabei Chloroquin, Amodiaquin, Mepacrin und Sontaquin eingesetzt.

Als weiterer bevorzugter aktiver Wirkstoff gegen Malaria oder anderer durch Plasmodien verursachter Infektion werden Antifolate eingesetzt. Hierzu zählen z.B. Pyrimethamin, Proguanil, Sulphone und Sulphonamide.

Für die resistenzfreie Therapie von Leishmaniasen werden als aktive Wirkstoffe bevorzugt Chemotherapeutika, Antibiotika und/oder Antiinfektiva eingesetzt. Als bevorzugtes Chemotherapeutikum wird dabei Methotrexat verwendet.

Die 5-substituierten Nukleoside bzw, deren Prodrugs werden bevorzugt in solchen Konzentrationen eingesetzt, daß nach der Verabreichung eine Konzentration der 5-substituierten Nukleoside bzw. deren Prodrugs im Blut zwischen 0,01 und 10 µg/mL, besonders bevorzugt zwischen 0,05 und 5 µg/mL, resultiert.

Die aktiven Wirkstoffe werden je nach Art der Infektionskrankheit in üblichen Konzentrationen verabreicht, wie sie z.B. in gängigen Arzneimittelverzeichnissen, aufgeführt sind. Hier sei besonders auf die Rote Liste 2001 (Rote Liste Service GmbH, Frankfurt/Main) verwiesen.

Die Verabreichung sowohl der aktiven Wirkstoffe als auch der 5-substituierten Nukleoside kann durch Injektion, oral, rektal, intravaginal, intranasal und/oder durch lokale Applikation erfolgen.

Neben dem aktiven Wirkstoff und den 5-substituierten Nukleosiden können als weitere Zusatzstoffe wäßrige und nicht-wäßrige Lösungsmittel, stabilisatoren, Suspensions-, Dispersions- und Benetzungsmittel in den Formulierungen eingesetzt werden. Als zusätzliche Additive kommen z.B. Polyethylenglykole, Farbstoffe und Parfümierungestoffe in Frage. Die Formulierung kann dabei in Form eines Pulvers, Puders, einer Suspension, einer Lösung, einer Emulsion, einer Salbe oder einer Paste erfolgen.

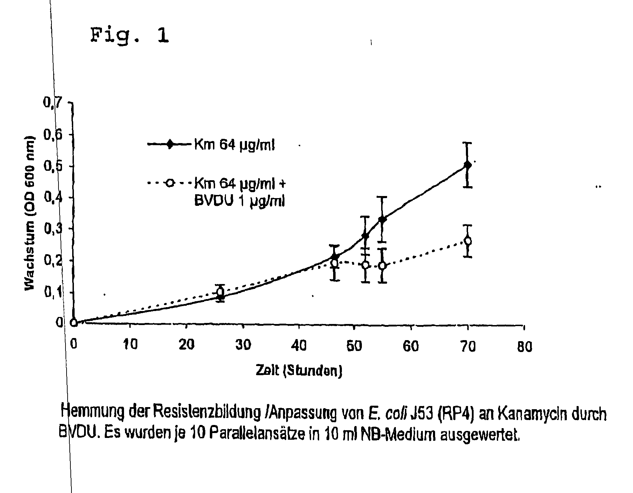

Anhand der folgenden Beispiele und der Figuren 1 bis 10 soll der erfindungsgemäße. Gegenstand näher erläutert werden, ohne diesen auf diese Beispiele zu beschränken.

### 1. Beispiel: Verhinderung der Resistenzbildung bei Bakterien

Zur Testung der Wirksamkeit von BVDU wurde Escherichia coli J53 als gramnegativer Modellorganismus verwendet. Dieser trägt das in der Natur vorkommende Plasmid RP4, auf dem Resistenzen gegenüber Ampicillin, Tetracyclin und Kanamycin codiert sind.

### 1.1 Anpassung an Kanamycin

Kanamycin ist ein natürlich vorkommendes Aminoglykosid-Antibiotikum. Es wird für die Behandlung bakterieller Infektionen eingesetzt, wenn es nicht möglich ist, Penicillin oder weniger toxische Antibiotika zu verwenden. Einsatzbereiche sind u.a. Infektionen der Knochen, der Atmungswege und der Haut sowie komplizierte Harnwegsinfektionen und Endokarditis.

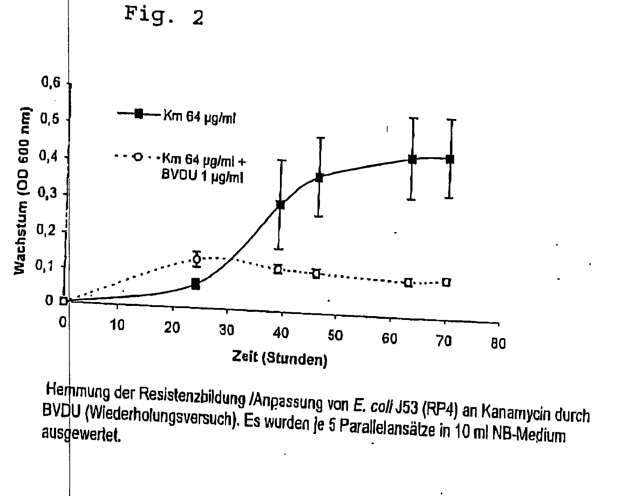

In Wachstumsversuchen mit Kanamycin (64 µg/ml) wurde eine langsame Anpassung, d.h. Resistenzbildung, von E. coli J53 (RP4) an diese Konzentration des Antibiotikums erreicht. In Gegenwart von BVDU (1 µg/ml) trat eine Hemmung dieser Resistenzbildung/Anpassung ein. Die Ergebnisse sind in den Figuren 1 und 2 dargestellt.

Die allmähliche Anpassung an 64 µg/ml Kanamycin führte zu einer stabilen Veränderung des Resistenzspektrums gegenüber dem eingesetzten Antibiotikum.

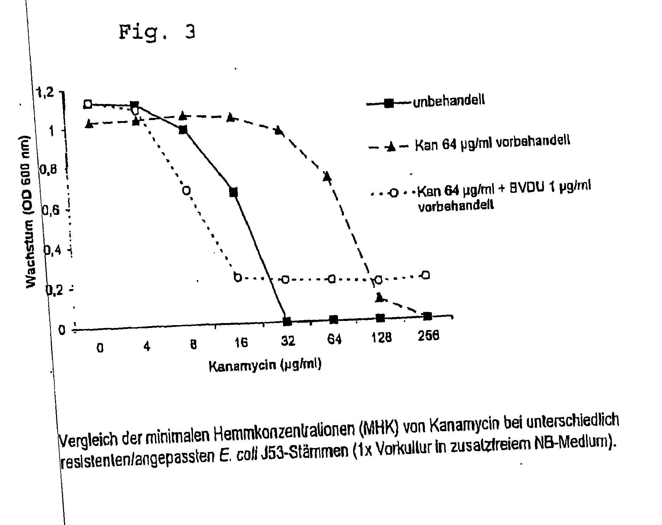

Für die Bestimmung der minimalen Hemmkonzentrationen (MHK) wurden zunächst Vorkulturen verwendet, für die Antibiotika-freiee Nährmedium direkt aus gefrorenen Glycerinkulturen beimpft worden war. Der gegenüber 64 µg/ml Kanamycin resistent gewordene Stamm wies mit 128 µg/ml eine 4fach erhöhte MHK gegenüber dem Ausgangsstamm auf (Fig. 3). Der in Gegenwart von 1 µg/ml BVDU gewachsene Stamm, der keine Resistenz gegenüber 64 µg/ml Kanamycin erreicht hatte, zeigte bereits ab 16 µg/ml Kanamycin Wachstumshemmung. Jedoch führten auch höhere Antibiotikum-Konzentrationen noch zum Auswachsen des Inokulums. Die Wachstumahemmung verhinderte aber das Erreichen einer größeren Zelldichte in der Bakterienkultur.

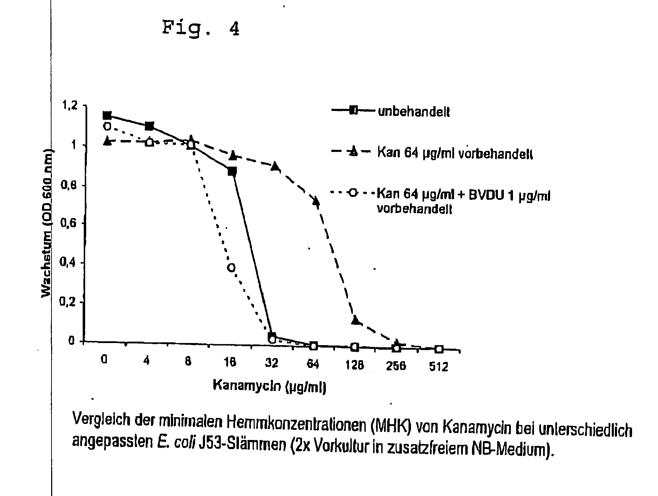

Zur Testung der Stabilität der spezifischen Resistenzmerkmale der E. coli-Stämme wurde die MHK-Bestimmung wiederholt. Die bereits getesteten Vorkulturen wurden dazu erneut in Antibiotika-freiem Nährmedium angezogen und auf ihre Kanamycin-Resistenz geprüft. Die Resistenzspektren des Ausgangsstammes und des gegenüber 64 µg/ml Kanamycin angepassten Stammes blieben unverändert. Unterschiede zeigten sich jedoch bei dem mit 64 µg/ml Kanamycin + 1 µg/ml BVDU vorbehandelten Stamm. Das Auswachsen des Inokulums wurde hierbei ab einer Konzentration von 32 µg/ml Kanamycin vollständig verhindert. Damit entsprach die MHK dieses Stammes der des Ausgangestammes. Die Ergebnisse sind in Figur 4 dargestellt.

Zusammenfassend bedeutet dies, dass BVDU die Resistenzbildung gegenüber Antibiotika verhindert und die Sensitivität gegenüber den Antibiotika in einem bestimmten Konzentrationsbereich erhöht.

### 1.2 Anpassung an Amikacin

Amikacin wirkt gegen Erreger, die gegen die übrigen Aminoglykoside resistent sind. Es wird bei schweren infektiösen Erkrankungen der Nieren, Harn- und Geschlechtsorgane und bei Infektionen der Atemwege und des Magen-Darm-Trakts gegeben.

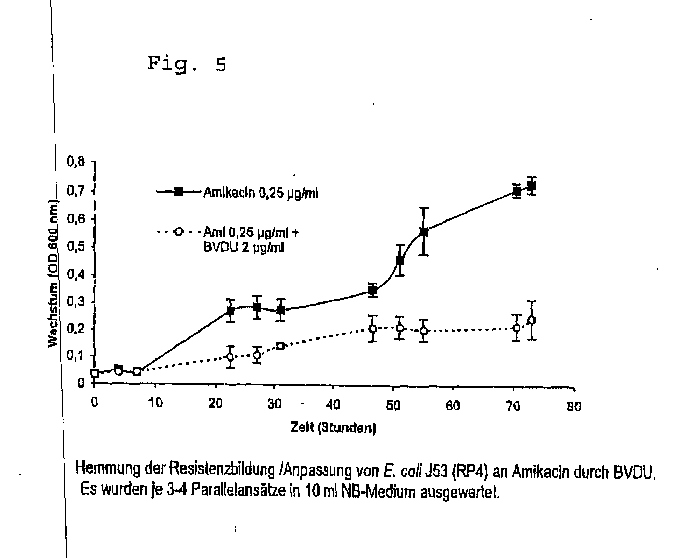

In Wachatumsversuchen mit Amikacin (0,25 µg/ml) wurde eine langsame Anpassung, d.h. Resistenzbildung, von E. coli J53 (RP4) an diese Konzentration des Antibiotikums erreicht. In Gegenwart von BVDU (2 µg/ml) trat eine Hemmung dieser Anpassung ein (Fig. 5).

Die allmähliche Anpassung an 0,25 µg/ml Amikacin führte zu einer stabilen Veränderung des Resistenzspektrums gegenüber dem eingesetzten Antibiotikum.

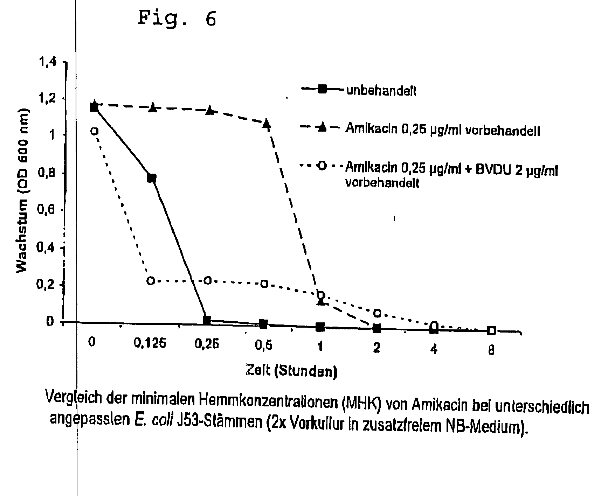

Der gegenüber 0,25 µg/ml Amikacin resistent gewordene Stamm wies mit 2 µg/ml eine 8fach erhöhte minimale Hemmkonzentration (MHK) gegenüber dem Ausgangsatamm auf. Der in Gegenwart von 2 µg/ml BVDU gewachsene Stamm, der keine Resistenz gegenüber 0,25 µg/ml Amikacin erreicht hatte, zeigte bereits ab 0,125 µg/ml Amikacin Wachstumshemmung. Diese Hemmung verhinderte auch bei höheren Antibiotikum-Konzentrationen das Erreichen einer größeren Zelldichte in der Bakterienkultur. Die Ergebnisse sind aus Figur 6 zu entnehmen.

### 2. Beispiel: BVDU-bedingte Wachstumshemmung nach Aminoglykosid/BVDU-Vorbehandlung

Zur Bestimmung der minimalen Hemmkonzentrationen (MHK) von Kanamycin bzw. Amikacin gegenüber Aminoglykosid/BVDU-vorbehandelten E. coli J53 (RP4)-Stämmen wurden standardmäßig Vorkulturen verwendet, die keine Zusätze im Anzuchtmedium enthielten (Erholungsphase). Daneben wurde in Vergleichsexperimenten getestet, inwieweit der Zusatz von BVDU zu den antibiotikafreien Vorkulturen das Resistenzspektrum der Stämme verändert.

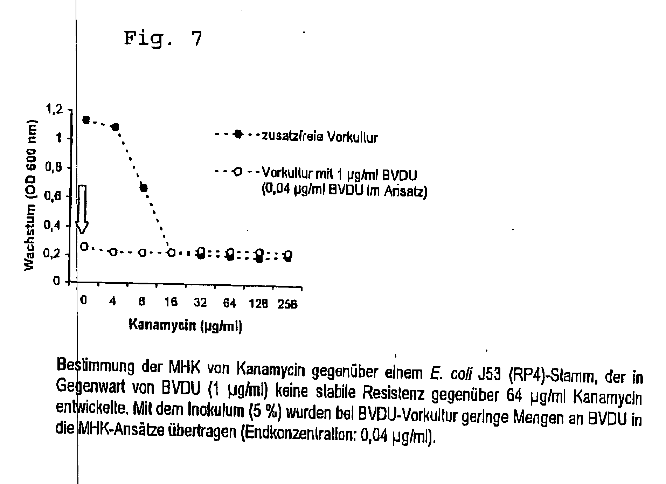

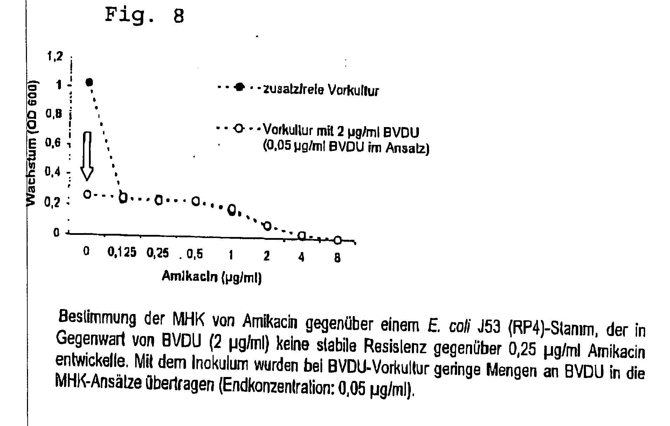

Die Anwesenheit von BVDU (1 bzw, 2 µg/ml) in der Vorkultur oder/und die durch Übertrag in den MHK-Ansätzen vorhandene geringe BVDU-Menge (0,04-0,05 µg/ml) reichten aus, um das Wachstum von Aminoglykosid/BVDUvorbehandelten E, coli-Stämmen auch in Abwesenheit des Antibiotikums nachhaltig einzuschränken. Im Vergleich dazu entsprach bei zusatzfreier Vorkultur das Wachstum ohne Antibiotikum dem des unbehandelten E. coli-Stammes. Die Ergebnisse sind in den Figuren 7 und 8 dargestellt.

Zusammenfassend bedeutet dies, dass BVDU nach Absetzen eines Antibiotikums auch allein wirkt. BVDU ohne Vorbehandlung (Antibiotikum + BVDU) entfaltet keine Wirkung und ist nicht toxisch.

### 3. Beispiel: Verhinderung der Resistenzbildung bei Protozoen

Verhinderung der Resistenzbildung bei Zooflagellaten (Leishmanien) durch die gleichzeitige Gabe des antirekombinogenen 5-substituierten Nukleoside (E)-5-(2-bromvvinyl)2'-deoxyuridine (BVDU) mit Methotrexat.

Infektiös sind auch Protozoen des Stammes Zooflagellata wie z.B. Trypanosoma als Erreger der Schlafkrankheit und Leishmanien (intrazellulär parasitierende Protozoen der Klasse Mastigophora) als Erreger der Leishmaniasen. Resistenzen gegenüber einer Chemotherapie beruhen sowohl bei Trypanosomen (Wilson et al., 1991) als auch Leiahmanien (Ouellette and Borst, 1991; Grondin et al., 1998; Arana et al., 1998; Haimeur and Ouellette, 1998; Kundig et al., 1999) auf der Amplifikation einiger Gene, die auch bei der Chemoresistenz von Tumoren eine Rolle spielen. Über die Verhinderung Chemotherapie-induzierter Rekombination/Genamplifikation soll die Resistenzbildung verhindert werden.

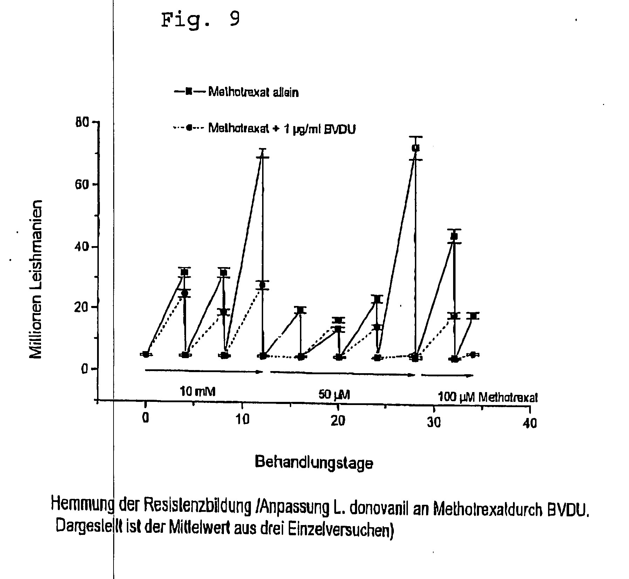

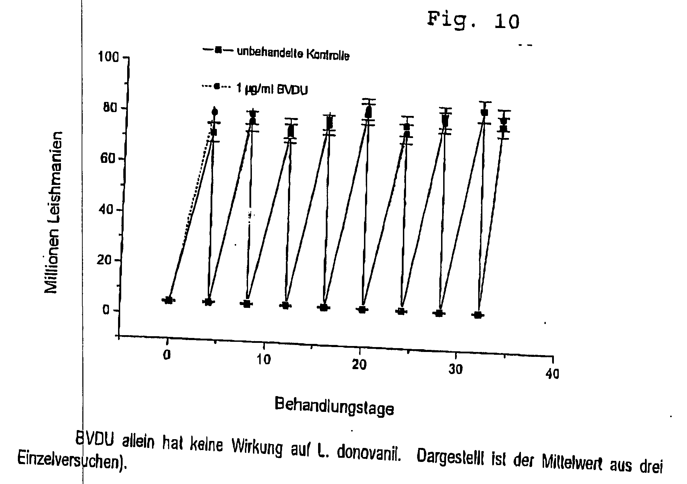

Methotrexat (MTX) resistente Zellen von Leishmania donovanii wurden durch stufenweise Erhöhung der MTX-Konzentrationen von 5 auf 10, von 10 auf 50 und von 50 auf 100 µM MTX erzeugt. Eingesät in das Wuchsmedium wurden jeweils 5 x 10⁶ Zellen/ml. Für jeden neuen Ansatz wurden die Zellen wieder auf 5 x 10⁶ Zellen/ml verdünnt. Die Zellen wurden immer dann der nächsthöheren MTX-Konzentration ausgesetzt, wenn sich die Zellteilungsrate der MTX-ausgesetzten Zellen auf Kontrollniveau stabilisiert hatte. Dies war nach jeweils etwa drei bis vier Passagen möglich. Setze man den Kulturen gleichzeitig 1 µg/ml BVDU zu, so erreichte die Zellteilungsrate niemals das Kontrollniveau, d.h., die Zellen entwickelten im Gegensatz zu den mit MTX allein behandelten Zellen keine Resistenz gegenüber der Behandlung. Vereinfacht ist das Ergebnis dieses Versuchs in den Figuren 9 und 10 zu sehen.

## Patentansprüche

1. Verwendung von 5-substituierten Nukleosiden ausgewählt aus (E)-5-(2-Bromovinyl)-2-deoxyuridine (BVDU), dessen Salzen, dessen Schutzformen und dessen Prodrugs. zusammen mit mindestens einem aktiven Wirkstoff zur Herstellung eines Arzneimittels oder Kombinationspräparats zur resistenzfreien Therapie von durch Bakterien oder Protozoen hervorgerufenen Infektionskrankheiten.

2. Verwendung nach Anspruch 1,
**dadurch gekennzeichnet, dass** 5-substituierte Nukleoside bzw. deren Prodrugs und der aktive Wirkstoff in einer einzigen Formulierung vorliegen.

3. Verwendung nach Anspruch 1,
**dadurch gekennzeichnet, dass** 5-substituierte Nukleoside bzw. deren Prodrugs und der aktive Wirkstoff in getrennten Formulierungen vorliegen.

4. Verwendung nach Anspruch 1,
**dadurch gekennzeichnet, dass** als Prodrug die Verbindung der Formel I eingesetzt wird

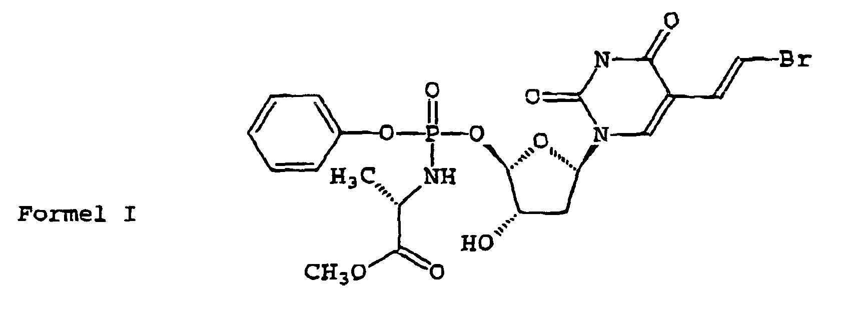

5. Verwendung nach einem der Ansprüche 1 bis 4,
**dadurch gekennzeichnet, dass** zur resistenzfreien Therapie bakterieller Infektionskrankheiten als aktive Wirkstoffe Antibiotika eingesetzt werden.

6. Verwendung nach Anspruch 5,
**dadurch gekennzeichnet, dass** die Bakterien ausgewählt sind aus der Gruppe der Proteusbakterien, Escherichia coli, Streptokokken und Staphylokokken.

7. Verwendung nach einem der Ansprüchen 1 bis 4,
**dadurch gekennzeichnet, dass** zur resistenzfreien Therapie von Malaria oder anderer durch Plasmodien verursachter Infektionen als aktive wirkstoffe Antibiotika und/oder Antiinfektiva wie z.B. 4-Aminochinoline eingesetzt werden.

8. Verwendung nach Anspruch 7,
**dadurch gekennzeichnet, dass** die Chinin-Derivate ausgewählt sind aus der Gruppe Chloroquin, Amodiaquin. Mepacrin und Sontaquin.

9. Verwendung nach einem der Ansprüche 1 bis 4,
**dadurch gekennzeichnet, dass** zur resistenzfreien Therapie von Malaria oder anderer durch Plasmodien verursachter Infektionen als aktive Wirkstoffe Antifolate eingesetzt werden.

10. Verwendung nach Anspruch 9,
**dadurch gekennzeichnet, dass** die Antifolate ausgewählt sind aus der Gruppe Pyrimethamin, Proguanil, Sulphone und Sulphonamide.

11. Verwendung nach einem der Ansprüche 1 bis 4,
**dadurch gekennzeichnet, dass** zur resistenzfreien Therapie von Leiahmaniasen als aktive Wirkstoffe Chemotherapeutika, Antibiotika und/oder Antiinfektiva eingesetzt werden.

12. Verwendung nach Anspruch 11,
**dadurch gekennzeichnet, dass** als Chemotherapeutikum Methotrexat eingesetzt wird.

13. Verwendung nach einem der Ansprüche 1 bis 12,
**dadurch gekennzeichnet, dass** die 5-substituierten Nukleoside bzw. deren Prodrugs in Konzentrationen eingesetzt werden, derart dass eine Blutkonzentration von 0,01 bis 10 µg/mL resultiert.

14. Verwendung nach Anspruch 13,
**dadurch gekennzeichnet, dass** die 5-substituierten Nukleoside bzw. deren Prodrugs in Konzentrationen eingesetzt werden, derart dass eine Blutkonzentration von 0,05 bis 5 µg/mL resultiert.

15. Verwendung nach einem der Ansprüche 1 bis 14,
**dadurch gekennzeichnet, dass** die aktiven Wirkstoffe in für die Infektionskrankheiten üblichen Konzentrationen eingesetzt werden.

16. Verwendung nach einem der Ansprüche 1 bis 15,
**dadurch gekennzeichnet, dass** die Verabreichung durch Injektion, oral, rektal, intravaginal, intranasal und/oder durch lokale Applikation erfolgt.

17. Verwendung nach einem der Ansprüche 1 bis 16,
**dadurch gekennzeichnet, dass** weitere Zusatzstoffe ausgewählt sind aus der Gruppe wäßrige und nicht-wäßrige Lösungsmittel, Stabilisatoren, Suspensions-, Dispersions- und Benetzungsmittel.

18. Verwendung nach einem der Ansprüche 1 bis 17,
**dadurch gekennzeichnet, dass** weitere Additive ausgewählt sind aus der Gruppe Polyethylenglykole, Farbstoffe und Parfümierungsstoffe.

19. Verwendung nach einem der Ansprüche 1 bis 18 in Form eines Pulvers, Puders, Suspension, Lösung, Emulsion, Salbe oder Paste.

## Claims

1. Use of 5-substituted nucleosides selected from (E)-5-(2-bromovinyl)-2-deoxyuridine (BVDU), its salts, its protected forms and its prodrugs, together with at least one active agent for producing a medicament or combination product for resistance-free therapy of infectious diseases caused by bacteria or protozoa.

2. Use according to Claim 1, **characterized in that** 5-substituted nucleosides or their prodrugs and the active agent are present in a single formulation.

3. Use according to Claim 1, **characterized in that** 5-substituted nucleosides or their prodrugs and the active agent are present in separate formulations.

4. Use according to Claim 1, **characterized in that** the compound of the formula I 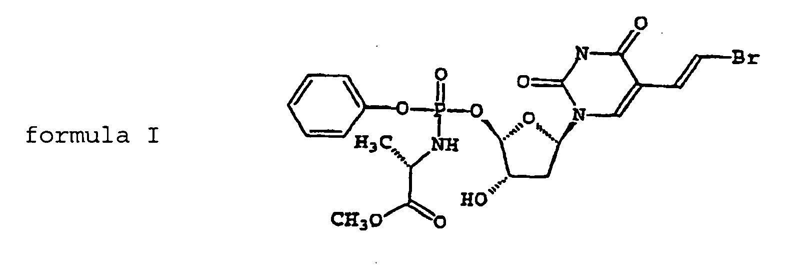 is employed as prodrug.

5. Use according to any of Claims 1 to 4, **characterized in that** antibiotics are employed as active agents for resistance-free therapy of bacterial infectious diseases.

6. Use according to Claim 5, **characterized in that** the bacteria are selected from the group of Proteus bacteria, Escherichia coli, streptococci and staphylococci.

7. Use according to any of Claims 1 to 4, **characterized in that** antibiotics and/or antiinfectives such as, for example, 4-aminoquinolines are employed as active agents for resistance-free therapy of malaria or other infections caused by plasmodias.

8. Use according to Claim 7, **characterized in that** the quinine derivatives are selected from the group of chloroquine, amodiaquine, mepacrine and sontaquine.

9. Use according to any of Claims 1 to 4, **characterized in that** antifolates are employed as active agents for resistance-free therapy of malaria or other infections caused by plasmodias.

10. Use according to Claim 9, **characterized in that** the antifolates are selected from the group of pyrimethamine, proguanil, sulphones and sulphonamides.

11. Use according to any of Claims 1 to 4, **characterized in that** chemotherapeutics, antibiotics and/or antiinfectives are employed as active agents for resistance-free therapy of leishmaniases.

12. Use according to Claim 11, **characterized in that** methotrexate is employed as chemotherapeutic.

13. Use according to any of Claims 1 to 12, **characterized in that** the 5-substituted nucleosides or their prodrugs are employed in concentrations such that a blood concentration of from 0.01 to 10 µg/ml results.

14. Use according to Claim 13, **characterized in that** the 5-substituted nucleosides or their prodrugs are employed in concentrations such that a blood concentration of from 0.05 to 5 µg/ml results.

15. Use according to any of Claims 1 to 14, **characterized in that** the active agent is employed in concentrations usual for infectious diseases.

16. Use according to any of Claims 1 to 15, **characterized in that** administration takes place by injection, orally, rectally, intravaginally, intranasally and/or by local application.

17. Use according to any of Claims 1 to 16, **characterized in that** further added substances are selected from the group of aqueous and nonaqueous solvents, stabilizers, suspending agents, dispersants and wetting agents.

18. Use according to any of Claims 1 to 17, **characterized in that** further additives are selected from the group of polyethylene glycols, colorants and perfuming substances.

19. Use according to any of Claims 1 to 18 in the form of an oral powder, dusting powder, suspension, solution, emulsion, ointment or paste.

## Revendications

1. Utilisation de nucléosides 5-substitués choisis parmi les (E)-5-(2-bromovinyl)-2-désoxyuridines (BVDU), leurs sels, leurs formes protégées et leurs promédicaments, conjointement avec au moins un principe actif, pour la fabrication d'un médicament ou d'une préparation mixte pour la thérapie sans résistance de maladies infectieuses provoquées par des bactéries ou des protozoaires.

2. Utilisation selon la revendication 1, **caractérisée en ce que** les nucléosides 5-substitués ou leurs promédicaments, et le principe actif, se présentent en une seule formulation.

3. Utilisation selon la revendication 1, **caractérisée en ce que** les nucléosides 5-substitués, ou leurs promédicaments, et le principe actif, se présentent dans des formulations séparées.

4. Utilisation selon la revendication 1, **caractérisée en ce que** l'on utilise comme promédicament le composé de formule I :

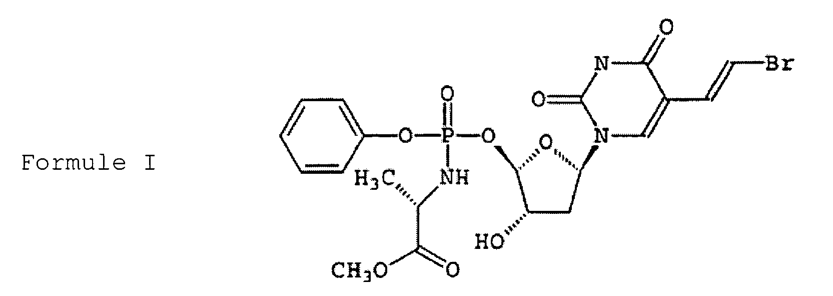

5. Utilisation selon l'une quelconque des revendications 1 à 4, **caractérisée en ce que** l'on utilise comme principes actifs des antibiotiques pour la thérapie sans résistance de maladies infectieuses bactériennes.

6. Utilisation selon la revendication 5, **caractérisée en ce que** les bactéries sont choisies dans le groupe des bactéries Proteus, de l'Escherichia coli, des streptocoques et des staphylocoques.

7. Utilisation selon l'une quelconque des revendications 1 à 4, **caractérisée en ce que** l'on utilise comme principes actifs des antibiotiques et/ou des anti-infectieux, par exemple, la 4-amino-quinoléine, pour la thérapie sans résistance de la malaria ou d'autres infections provoquées par les plasmodies.

8. Utilisation selon la revendication 7, **caractérisée en ce que** les dérivés de la quinine sont choisis dans le groupe de la chloroquine, de l'amodiaquine, de la mépacrine et de la sontaquine.

9. Utilisation selon l'une quelconque des revendications 1 à 4, **caractérisée en ce que** l'on utilise comme principes actifs des antifolates pour la thérapie sans résistance de la malaria ou d'autres infections provoquées par les plasmodies.

10. Utilisation selon la revendication 9, **caractérisée en ce que** les antifolates sont choisis dans le groupe de la pyriméthamine, du proguanil, des sulfones et des sulfonamides.

11. Utilisation selon l'une quelconque des revendications 1 à 4, **caractérisée en ce que** l'on utilise comme principes actifs des chimio-thérapeutiques, des antibiotiques et/ou des anti-infectieux pour la thérapie sans résistance de la leishmaniose.

12. Utilisation selon la revendication 11, **caractérisée en ce que** l'on utilise du méthotrexate comme chimiothérapeutique.

13. Utilisation selon l'une quelconque des revendications 1 à 12, **caractérisée en ce que** l'on utilise les nucléosides 5-substitués ou leurs promédicaments en concentrations telles que l'on obtienne une concentration dans le sang de 0,01 à 10 µg/µL.

14. Utilisation selon la revendication 13, **caractérisée en ce que** l'on utilise les nucléosides 5-substitués ou leurs promédicaments en concentrations telles que l'on obtienne une concentration dans le sang de 0,05 à 5 µg/µL.

15. Utilisation selon l'une quelconque des revendications 1 à 14, **caractérisée en ce que** l'on utilise les principes actifs en concentrations usuelles pour les maladies infectieuses.

16. Utilisation selon l'une quelconque des revendications 1 à 15, **caractérisée en ce que** l'administration se fait par injection, par voie orale, rectale, intravaginale, intranasale et/ou par application locale.

17. Utilisation selon l'une quelconque des revendications 1 à 16, **caractérisée en ce que** d'autres adjuvants sont choisis dans le groupe des solvants aqueux ou non-aqueux, des stabilisateurs, des agents de suspension, des agents de dispersion et des agents mouillants.

18. Utilisation selon l'une quelconque des revendications 1 à 17, **caractérisée en ce que** d'autres additifs sont choisis dans le groupe des polyéthylène-glycols, des colorants et des parfums.

19. Utilisation selon l'une quelconque des revendications 1 à 18 sous la forme d'une poudre, d'une suspension, d'une solution, d'une émulsion, d'une pommade ou d'une pâte.
